# EUROPEAN PATENT SPECIFICATION

(11) **EP 2 694 088 B1**
(45) Date of publication and mention of the grant of the patent: **07.06.2017**
(21) Application number: 12768091.6
(22) Date of filing: 05.04.2012
(51) Int. Cl.: A61K 38/14, A61P 31/18, A61P 35/00

(54) **MACROPHAGE ACTIVATING FACTOR FOR PHARMACEUTICAL COMPOSITIONS**
MAKROPHAGENAKTIVIERUNGSFAKTOR FÜR PHARMAZEUTISCHE ZUSAMMENSETZUNGEN
FACTEUR D'ACTIVATION DES MACROPHAGES POUR DES COMPOSITIONS PHARMACEUTIQUES

(30) Priority: 07.04.2011 US 201161472642 P
(43) Date of publication of application: 12.02.2014
(73) Proprietor: Efranat Ltd., 76701 Rehovot (IL)
(72) Inventor: YAMAMOTO, Nobuto, Philadelphia, Pennsylvania 10126-3305 (US)
(74) Representative: Becker Kurig Straus
(86) International application number: PCT/IL2012/000159
(87) International publication number: WO 2012/137199

(56) References cited:
- US-A- 5 326 749
- US-A1- 2003 036 638
- US-A1- 2005 261 172
- US-A1- 2007 275 001
- US-A1- 2009 041 793
- US-B1- 6 410 269
- NAGASAWA H ET AL.: "Gc Protein (Vitamin D-binding Protein): Gc Genotyping and GcMAF Precursor Activity", ANTICANCER RESEARCH, vol. 25, 1 January 2005 (2005-01-01), pages 3689-3696, XP055094333,
- NOBUTO YAMAMOTO ET AL: "Immunotherapy of HIV-infected patients with Gc protein-derived macrophage activating factor (GcMAF)", JOURNAL OF MEDICAL VIROLOGY, vol. 81, no. 1, 1 January 2009 (2009-01-01), pages 16-26, XP055010289, ISSN: 0146-6615, DOI: 10.1002/jmv.21376
- ZHAOLONG ET AL.: 'Isolation and Purification of Gc Protein by Fast Performance Liquid Chromatography System' CHINESE JOURNAL OF FORENSIC MEDICINE, [Online] vol. 11, no. 3, 02 March 1996, pages 135 - 138, XP008171062 Retrieved from the Internet: <URL:http://en.cnki.com.cn/Article_en/CJFDT OTAL- FUAN199603002.htm>
- SHELDON.: 'Enzyme Immobilization: The Quest for Optimum Performance' ADVANCED SYNTHESIS & CATALYSIS vol. 349, 04 June 2007, pages 1289 - 1307, XP055001404
- BEAURANG.: 'GE Healthcase MonoQ HR 5/5 Column', [Online] 29 July 2000, pages 1 - 2., XP008171068 Retrieved from the Internet: <URL:http://www.biocompare.com/Product-Revi ews/ 40365-GE-Healthcare-MonoQ-HR-5-5-column>
- YAMAMOTO ET AL.: 'Immunotherapy for Prostate Cancer with Gc Protein-Derived Macrophage-Activating Factor, GcMAF' TRANSLATIONAL ONCOLOGY vol. 1, no. 2, 01 July 2008, pages 65 - 72, XP008154499
- NAGASAWA ET AL.: 'Gc Protein (Vitamin D-binding Protein): Gc Genotyping and GcMAF Precursor Activity' ANTICANCER RESEARCH vol. 25, no. ISS.2A, 15 November 2005, pages 3689 - 3695, XP055094333

### SEQUENCE LISTING

<110> Efranat Ltd
<120> MACROPHAGE ACTIVATING FACTOR FOR PHARMACEUTICAL COMPOSITIONS
<130> NY/001 PCT
<150> US 61/472642
   <151> 2011-04-07
<160> 5
<170> PatentIn version 3.5
<210> 1
   <211> 458
   <212> PRT
   <213> Homo sapiens
<220>
   <221> MISC_FEATURE
   <222> (152)..(152)
   <223> X At position 152 may be Glycine or Glutamate
<220>
   <221> MISC_FEATURE
   <222> (429)..(429)
   <223> X At position 429 may be Arginine or Histidine
<400> 1
   Leu Glu Arg Gly Arg Asp Tyr Glu Lys Asn Lys Val Cys Lys Glu Phe
   1               5                   10                  15
   Ser His Leu Gly Lys Glu Asp Phe Thr Ser Leu Ser Leu Val Leu Tyr
               20                  25                  30
   Ser Arg Lys Phe Pro Ser Gly Thr Phe Glu Gln Val Ser Gln Leu Val
           35                  40                  45
   Lys Glu Val Val Ser Leu Thr Glu Ala Cys Cys Ala Glu Gly Ala Asp
       50                  55                  60
   Pro Asp Cys Tyr Asp Thr Arg Thr Ser Ala Leu Ser Ala Lys Ser Cys
   65                  70                  75                  80
   Glu Ser Asn Ser Pro Phe Pro Val His Pro Gly Thr Ala Glu Cys Cys
                   85                  90                  95
   Thr Lys Glu Gly Leu Glu Arg Lys Leu Cys Met Ala Ala Leu Lys His
               100                 105                 110
   Gln Pro Gln Glu Phe Pro Thr Tyr Val Glu Pro Thr Asn Asp Glu Ile
           115                 120                 125
   Cys Glu Ala Phe Arg Lys Asp Pro Lys Glu Tyr Ala Asn Gln Phe Met
       130                 135                 140
   Trp Glu Tyr Ser Thr Asn Tyr Xaa Gln Ala Pro Leu Ser Leu Leu Val
   145                 150                 155                 160
   Ser Tyr Thr Lys Ser Tyr Leu Ser Met Val Gly Ser Cys Cys Thr Ser
                   165                 170                 175
   Ala Ser Pro Thr Val Cys Phe Leu Lys Glu Arg Leu Gln Leu Lys His
               180                 185                 190
   Leu Ser Leu Leu Thr Thr Leu Ser Asn Arg Val Cys Ser Gln Tyr Ala
           195                 200                 205
   Ala Tyr Gly Glu Lys Lys Ser Arg Leu Ser Asn Leu Ile Lys Leu Ala
       210                 215                 220
   Gln Lys Val Pro Thr Ala Asp Leu Glu Asp Val Leu Pro Leu Ala Glu
   225                 230                 235                 240
   Asp Ile Thr Asn Ile Leu Ser Lys Cys Cys Glu Ser Ala Ser Glu Asp
                   245                 250                 255
   Cys Met Ala Lys Glu Leu Pro Glu His Thr Val Lys Leu Cys Asp Asn
               260                 265                 270
   Leu Ser Thr Lys Asn Ser Lys Phe Glu Asp Cys Cys Gln Glu Lys Thr
           275                 280                 285
   Ala Met Asp Val Phe Val Cys Thr Tyr Phe Met Pro Ala Ala Gln Leu
       290                 295                 300
   Pro Glu Leu Pro Asp Val Glu Leu Pro Thr Asn Lys Asp Val Cys Asp
   305                 310                 315                 320
   Pro Gly Asn Thr Lys Val Met Asp Lys Tyr Thr Phe Glu Leu Ser Arg
                   325                 330                 335
   Arg Thr His Leu Pro Glu Val Phe Leu Ser Lys Val Leu Glu Pro Thr
               340                 345                 350
   Leu Lys Ser Leu Gly Glu Cys Cys Asp Val Glu Asp Ser Thr Thr Cys
           355                 360                 365
   Phe Asn Ala Lys Gly Pro Leu Leu Lys Lys Glu Leu Ser Ser Phe Ile
           370                 375                 380
   Asp Lys Gly Gln Glu Leu Cys Ala Asp Tyr Ser Glu Asn Thr Phe Thr
   385                 390                 395                 400
   Glu Tyr Lys Lys Lys Leu Ala Glu Arg Leu Lys Ala Lys Leu Pro Asp
                   405                 410                 415
   Ala Thr Pro Thr Glu Leu Ala Lys Leu Val Asn Lys Xaa Ser Asp Phe
               420                 425                 430
   Ala Ser Asn Cys Cys Ser Ile Asn Ser Pro Pro Leu Tyr Cys Asp Ser
           435                 440                 445
   Glu Ile Asp Ala Glu Leu Lys Asn Ile Leu
       450                 455
<210> 2
   <211> 458
   <212> PRT
   <213> Homo sapiens
<220>
   <221> MISC_FEATURE
   <222> (152)..(152)
   <223> X at position 152 may be Glycine or Glutamate
<220>
   <221> MISC_FEATURE
   <222> (429)..(429)
   <223> X at position 429 may be Arginine or Histidine
<400> 2
   Leu Glu Arg Gly Arg Asp Tyr Glu Lys Asn Lys Val Cys Lys Glu Phe
   1               5                   10                  15
   Ser His Leu Gly Lys Glu Asp Phe Thr Ser Leu Ser Leu Val Leu Tyr
               20                  25                  30
   Ser Arg Lys Phe Pro Ser Gly Thr Phe Glu Gln Val Ser Gln Leu Val
           35                  40                  45
   Lys Glu Val Val Ser Leu Thr Glu Ala Cys Cys Ala Glu Gly Ala Asp
       50                  55                  60
   Pro Asp Cys Tyr Asp Thr Arg Thr Ser Ala Leu Ser Ala Lys Ser Cys
   65                  70                  75                  80
   Glu Ser Asn Ser Pro Phe Pro Val His Pro Gly Thr Ala Glu Cys Cys
                   85                  90                  95
   Thr Lys Glu Gly Leu Glu Arg Lys Leu Cys Met Ala Ala Leu Lys His
             100                 105                 110
   Gln Pro Gln Glu Phe Pro Thr Tyr Val Glu Pro Thr Asn Asp Glu Ile
         115                 120                 125
   Cys Glu Ala Phe Arg Lys Asp Pro Lys Glu Tyr Ala Asn Gln Phe Met
       130                 135                 140
   Trp Glu Tyr Ser Thr Asn Tyr Xaa Gln Ala Pro Leu Ser Leu Leu Val
   145                 150                 155                 160
   Ser Tyr Thr Lys Ser Tyr Leu Ser Met Val Gly Ser Cys Cys Thr Ser
                   165                 170                 175
   Ala Ser Pro Thr Val Cys Phe Leu Lys Glu Arg Leu Gln Leu Lys His
               180                 185                 190
   Leu Ser Leu Leu Thr Thr Leu Ser Asn Arg Val Cys Ser Gln Tyr Ala
           195                 200                 205
   Ala Tyr Gly Glu Lys Lys Ser Arg Leu Ser Asn Leu Ile Lys Leu Ala
       210                 215                 220
   Gln Lys Val Pro Thr Ala Asp Leu Glu Asp Val Leu Pro Leu Ala Glu
   225                 230                 235                 240
   Asp Ile Thr Asn Ile Leu Ser Lys Cys Cys Glu Ser Ala Ser Glu Asp
                   245                 250                 255
   Cys Met Ala Lys Glu Leu Pro Glu His Thr Val Lys Leu Cys Asp Asn
               260                 265                 270
   Leu Ser Thr Lys Asn Ser Lys Phe Glu Asp Cys Cys Gln Glu Lys Thr
           275                 280                 285
   Ala Met Asp Val Phe Val Cys Thr Tyr Phe Met Pro Ala Ala Gln Leu
       290                 295                 300
   Pro Glu Leu Pro Asp Val Glu Leu Pro Thr Asn Lys Asp Val Cys Asp
   305                 310                 315                 320
   Pro Gly Asn Thr Lys Val Met Asp Lys Tyr Thr Phe Glu Leu Ser Arg
                   325                 330                 335
   Arg Thr His Leu Pro Glu Val Phe Leu Ser Lys Val Leu Glu Pro Thr
                   340                 345                 350
   Leu Lys Ser Leu Gly Glu Cys Cys Asp Val Glu Asp Ser Thr Thr Cys
           355                 360                 365
   Phe Asn Ala Lys Gly Pro Leu Leu Lys Lys Glu Leu Ser Ser Phe Ile
       370                 375                 380
   Asp Lys Gly Gln Glu Leu Cys Ala Asp Tyr Ser Glu Asn Thr Phe Thr
   385                 390                 395                 400
   Glu Tyr Lys Lys Lys Leu Ala Glu Arg Leu Lys Ala Lys Leu Pro Glu
                   405                 410                 415
   Ala Thr Pro Thr Glu Leu Ala Lys Leu Val Asn Lys Xaa Ser Asp Phe
               420                 425                 430
   Ala Ser Asn Cys Cys Ser Ile Asn Ser Pro Pro Leu Tyr Cys Asp Ser
           435                 440                 445
   Glu Ile Asp Ala Glu Leu Lys Asn Ile Leu
       450                 455
<210> 3
   <211> 458
   <212> PRT
   <213> Homo sapiens
<220>
   <221> MISC_FEATURE
   <222> (152)..(152)
   <223> X at position 152 may be Glycine or Glutamate
<220>
   <221> MISC_FEATURE
   <222> (429)..(429)
   <223> X at position 429 may be Arginine or Histidine
<400> 3
   Leu Glu Arg Gly Arg Asp Tyr Glu Lys Asn Lys Val Cys Lys Glu Phe
   1               5                   10                  15
   Ser His Leu Gly Lys Glu Asp Phe Thr Ser Leu Ser Leu Val Leu Tyr
               20                  25                  30
   Ser Arg Lys Phe Pro Ser Gly Thr Phe Glu Gln Val Ser Gln Leu Val
           35                  40                  45
   Lys Glu Val Val Ser Leu Thr Glu Ala Cys Cys Ala Glu Gly Ala Asp
       50                  55                  60
   Pro Asp Cys Tyr Asp Thr Arg Thr Ser Ala Leu Ser Ala Lys Ser Cys
   65                  70                  75                  80
   Glu Ser Asn Ser Pro Phe Pro Val His Pro Gly Thr Ala Glu Cys Cys
                   85                  90                  95
   Thr Lys Glu Gly Leu Glu Arg Lys Leu Cys Met Ala Ala Leu Lys His
               100                 105                 110
   Gln Pro Gln Glu Phe Pro Thr Tyr Val Glu Pro Thr Asn Asp Glu Ile
           115                 120                 125
   Cys Glu Ala Phe Arg Lys Asp Pro Lys Glu Tyr Ala Asn Gln Phe Met
       130                 135                 140
   Trp Glu Tyr Ser Thr Asn Tyr Xaa Gln Ala Pro Leu Ser Leu Leu Val
   145                 150                 155                 160
   Ser Tyr Thr Lys Ser Tyr Leu Ser Met Val Gly Ser Cys Cys Thr Ser
                   165                 170                 175
   Ala Ser Pro Thr Val Cys Phe Leu Lys Glu Arg Leu Gln Leu Lys His
               180                 185                 190
   Leu Ser Leu Leu Thr Thr Leu Ser Asn Arg Val Cys Ser Gln Tyr Ala
           195                 200                 205
   Ala Tyr Gly Glu Lys Lys Ser Arg Leu Ser Asn Leu Ile Lys Leu Ala
       210                 215                 220
   Gln Lys Val Pro Thr Ala Asp Leu Glu Asp Val Leu Pro Leu Ala Glu
   225                 230                 235                 240
   Asp Ile Thr Asn Ile Leu Ser Lys Cys Cys Glu Ser Ala Ser Glu Asp
                   245                 250                 255
   Cys Met Ala Lys Glu Leu Pro Glu His Thr Val Lys Leu Cys Asp Asn
               260                 265                 270
   Leu Ser Thr Lys Asn Ser Lys Phe Glu Asp Cys Cys Gln Glu Lys Thr
           275                 280                 285
   Ala Met Asp Val Phe Val Cys Thr Tyr Phe Met Pro Ala Ala Gln Leu
       290                 295                 300
   Pro Glu Leu Pro Asp Val Glu Leu Pro Thr Asn Lys Asp Val Cys Asp
   305 310 315 320
   Pro Gly Asn Thr Lys Val Met Asp Lys Tyr Thr Phe Glu Leu Ser Arg
   325 330 335
   Arg Thr His Leu Pro Glu Val Phe Leu Ser Lys Val Leu Glu Pro Thr
   340 345 350
   Leu Lys Ser Leu Gly Glu Cys Cys Asp Val Glu Asp Ser Thr Thr Cys
   355 360 365
   Phe Asn Ala Lys Gly Pro Leu Leu Lys Lys Glu Leu Ser Ser Phe Ile
   370 375 380
   Asp Lys Gly Gln Glu Leu Cys Ala Asp Tyr Ser Glu Asn Thr Phe Thr
   385 390 395 400
   Glu Tyr Lys Lys Lys Leu Ala Glu Arg Leu Lys Ala Lys Leu Pro Asp
   405 410 415
   Ala Thr Pro Lys Glu Leu Ala Lys Leu Val Asn Lys Xaa Ser Asp Phe
   420 425 430
   Ala Ser Asn Cys Cys Ser Ile Asn Ser Pro Pro Leu Tyr Cys Asp Ser
   435 440 445
   Glu Ile Asp Ala Glu Leu Lys Asn Ile Leu
   450 455
<210> 4
   <211> 83
   <212> PRT
   <213> Artificial Sequence
<220>
   <223> Synthetic Peptide
<220>
   <221> misc_feature
   <222> (55)..(55)
   <223> Xaa can be any naturally occurring amino acid
<400> 4
   Leu Leu Lys Lys Glu Leu Ser Ser Phe Ile Asp Lys Gly Gln Glu Leu
   1               5                   10                  15
   Cys Ala Asp Tyr Ser Glu Asn Thr Phe Thr Glu Tyr Lys Lys Lys Leu
               20                  25                  30
   Ala Glu Arg Leu Lys Ala Lys Leu Pro Asp Ala Thr Pro Thr Glu Leu
           35                  40                  45
   Ala Lys Leu Val Asn Lys Xaa Ser Asp Phe Ser Asn Cys Cys Ser Ile
       50                  55                  60
   Asn Ser Pro Pro Leu Tyr Cys Asp Ser Glu Ile Asp Ala Glu Leu Lys
   65                  70                  75                  80
   Asn Ile Leu
<210> 5
   <211> 84
   <212> PRT
   <213> Artificial Sequence
<220>
   <223> Synthetic Peptide
<220>
   <221> misc_feature
   <222> (55)..(55)
   <223> Xaa can be any naturally occurring amino acid
<400> 5
   Leu Leu Lys Lys Glu Leu Ser Ser Phe Ile Asp Lys Gly Gln Glu Leu
   1               5                   10                  15
   Cys Ala Asp Tyr Ser Glu Asn Thr Phe Thr Glu Tyr Lys Lys Lys Leu
               20                  25                  30
   Ala Glu Arg Leu Lys Ala Lys Leu Pro Asp Ala Thr Pro Lys Glu Leu
           35                  40                  45
   Ala Lys Leu Val Asn Lys Xaa Ser Asp Phe Ala Ser Asn Cys Cys Ser
       50                  55                  60
   Ile Asn Ser Pro Pro Leu Tyr Cys Asp Ser Glu Ile Asp Ala Glu Leu
   65                  70                  75                  80
   Lys Asn Ile Leu

## Description

### FIELD OF THE INVENTION

The present invention relates to pharmaceutical compositions comprising Gc protein-derived macrophage activating factor (GcMAF) and method of producing same, particularly to GcMAF compositions essentially devoid of glycosidase enzymes.

### BACKGROUND OF THE INVENTION

Inflammation results in macrophage activation, leading to a progress in the immune response process. Inflammation-derived macrophage activation requires participation of B and T lymphocytes and serum vitamin D binding protein (DBP).

The human vitamin D-binding protein, also known as "group-specific component" or "Gc protein", is an evolutionary conserved glycoprotein showing genetic polymorphism. It is a plasma protein having a relative molecular weight of about 52,000, normally constituting about 0.5% of the plasma proteins in human. Polymorphism of the Gc protein is demonstrable by gel electrophoresis analysis, which reveals two major phenotypes: Gc1 and Gc2 (Hirschfeld et al., 1960. Nature 185:931). The entire nucleotide coding sequences of the Gc1 and Gc2 genes, and the predicted amino acid sequences have been reported (Cooke, et al., 1985. J. Clin. Invest. 76:2420; Yang et al., 1985. Proc. Natl. Acad. Sci. USA 82:7994). Gc1 is further divided into Gc1f and Gc1s subtypes, which migrate electrophoretically as two bands ("fast" and 'slow"), due to a variation in one amino acid residue.

Gc1 protein is the major subtype of human Gc protein. It carries a branched trisaccharide composed of N-acetylgalactosamine (GalNAc) attached to the core protein with a termini of galactose and sialic acid (in Gc1f) or galactose and mannose (in Gc1s). Gc2 has a simple glycosylation pattern with a core GalNAc linked to a terminal galactose moiety. Gc1f oligosaccharide is hydrolyzed by membranous β- galactosidase of inflammation-primed B cells to yield a macrophage pro-activating factor, which is in turn hydrolyzed by sialidase (also known as neuraminidase) of T-cells to yield a macrophage activating factor (MAF) (Yamamoto et al., 1991. Proc. Nail. Acad. Sci. USA 88:8539-8543; Yamamoto and Kumashiro 1993. Immunol, 151:2794-2902; Naraparaju and Yamamoto 1994. Immunol. Lets 43:143-148). Mouse DBP carries a disaccharide composed of N-acetylgalactosamine with a galactose terminal. Hydrolysis of this disaccharide by β-galactosidase of B cells alone generates a potent MAF. Thus, mouse DBP and human Gc protein are precursors for MAFs.

U.S. Patent No. 5,177,002 to the inventor of the present invention discloses a process for *in vitro* production of potent macrophage activating factor by treating the glycosylated vitamin D-binding protein with glycosidases. The phenotypes of Gc protein Gc1 and Gc2, and the Gc1 subtypes Gc1f and Gc1s, are expressed *inter alia* as differences in the oligosaccharides attached to the polypeptide portion of the Gc molecule. The macrophage activating factor is efficiently produced from Gc1f or Gc1s protein by incubation with a combination of β-galactosidase and sialidase (also known as neuraminidase), or a combination of β-galactosidase and α-mannosidase. If the Gc1s comprises at least in part the Gc1s variant, Gc1s*, which contains sialic acid (N-acetyl-D-neuramic acid, or "NeuNAc") in lieu of α-mannose, the mixture of enzymes utilized to treat the Gc1s/Gc1s* mixture also includes sialidase. Treatment of the Gc2 protein with β-galactosidase alone efficiently yields the macrophage activating factor. Thus, the 5,177,002 Patent discloses efficient conversion of Gc protein to the macrophage activating factor *in vitro,* in the absence of intact B- and T-cells, resulting in a highly potent factor designated GcMAF.

The uncontrolled growth of metastases resistant to conventional therapeutic modalities is a major cause of death from cancer. Metastases arise from the nonrandom spread of specialized malignant cells that preexist within a primary neoplasm. These metastases can be clonal in their origin, and different metastases can originate from different progenitor cells. In addition, metastatic cells can exhibit an increased rate of spontaneous mutations compared with benign non-metastatic cells, which explain the clinical observation that multiple metastases can exhibit different sensitivity to the same therapeutic modalities. A successful therapy of disseminated metastases thus should circumvent the problems of neoplastic heterogeneity and development of resistance.

Appropriately activated macrophages can fulfill these demanding criteria. Macrophages can be activated to become tumoricidal by interaction with phospholipid vesicles (liposomes) containing immunomodulators. Tumoricidal macrophages can recognize and destroy neoplastic cells in vitro and in vivo, leaving non-neoplastic cells uninjured. Although the exact mechanism(s) by which macrophages discriminate between tumorigenic and normal cells is unknown, it is independent of tumor cell characteristics such as immunogenicity, metastatic potential, and sensitivity to cytotoxic drugs. Moreover, macrophage destruction of tumor cells apparently is not associated with the development of tumor cell resistance. Additionally, activated macrophages are essential for the immune response to form relative to bacterial and viral invasion. As the mechanism of activation is identical in the three responses (tumoricidal, bactericidal, viricidal), the activation of macrophages has applications across the host immune response, against tumor, bacteria and viral challenges.

Studies have shown that GcMAF has a tumoricidal role in the treatment of an Ehrlich ascites tumor in a mouse model (Yamamoto et al., 1997. Cancer Res. 57:2187-2192; Koga et al., 1999. Proc Soc Exp Biol Med. 220:20-26). In a mice model of squamous cell carcinoma, administration of GcMAF as an adjuvant immunotherapy to photodynamic therapy showed a synergistic effect on tumor cure in mice (Korbelik et al. 1997.Br J Cancer 75:202-207). In both tumor models, it was hypothesized that GcMAF elicited its effect by activating macrophages, which then directly attacked the tumor cells. Further evidence suggested that GcMAF is anti-tumorigenic in part through an antiangiogenic mechanism (Kisker et al., 2003. Neoplasia 5(1):32-40). In all models, high potency of GcMAF as an anti-tumorigenic therapy was observed.

U.S. Patent Application Publication No. 2011/0123591 discloses methods of inducing a tumoricidal, bactericidal or viricidal response in a mammal by macrophage activation through the use of an extracorporeal system. The system comprises means for contacting a leukocyte fraction of the mammal's blood with GcMAF or with one or more enzymes that create endogenous GcMAF from Gc protein precursor. Alternatively, the system comprises alpha-N-acetylgalactosaminidase (Nagalase)-binding ligand immobilized on an inert medium that is contacted with the mammal's plasma, thus reducing the level of Nagalase.

The precursor of MAF, the glycosylated Gc protein, can be purified from blood source. Alternatively, Gc protein or its small domain responsible for macrophage activation can be produced employing recombinant methods, as disclosed, for example, in U.S. Patent No. 6,410,269 to the inventor of the present invention. Independent of the Gc protein source, it should be partially deglycosylated in order to obtain the macrophage activating factor as described hereinabove. The 5,177,002 Patent discloses the use of immobilized enzymes and passing the reaction mixture through an appropriate cut-off filter to avoid contamination of the final preparations with glycosidases. No particular guidelines are provided for selecting the immobilization means, with activated agarose beads being the preferred embodiments.

Nagasawa et al. (2005. Anticancer Res. 25:3689-3696) discloses that the Gc protein has important physiological functions, including involvement in vitamin D transport and storage, scavenging extracellular G-actin, and macrophage activation, the latter is mediated by GcMAF. In order to produce GcMAF, human serum was purified on 25-hydroxyvitamin D3 affinity chromatography to obtain Gc protein, which was then treated with immobilized β-galactosidase and sialidase. GcMAF displayed potent macrophage activation in an in vitro assay of mouse peritoneal macrophages.

Yamamoto et al. (2009. J. med. Virol. 81: 16-26) discloses immunotherapy of HIV-infected patients with GcMAF. The GcMAF was produced by purification of Gc protein using 25-hydroxyvitamin D3 affinity chromatography, followed by stepwise treatment of the purified Gc protein with immobilized β-galactosidase and sialidase. The GcMAF produced was claimed to be the most potent macrophage activating factor ever discovered.

US 5,326,749 discloses the production of macrophage activating factor from glycosylated vitamin D-binding protein (DBP) isolated from blood or plasma of mammals. The conversion of DBP to macrophage activating factor was performed by using sialidase in insoluble form, e.g., attached to agarose beads, to avoid contamination of the resulting macrophage activating factor with sialidase fragments. The macrophage activating factor was then produced by adding β-galactosidase in a liquid medium, followed by subsequent filtration of the liquid on 100 kDa cut off filter to recover the macrophage activating factor and to remove the immobilized sialidase and β-galactosidase.

As of today, the use of GcMAF has been limited to *ex vivo* treatments or to intramuscular administrations in order to avoid adverse side effects, apparently due to the presence of contaminating proteins in the compositions produced as per the procedures previously disclosed.

As the potential of GcMAF as a potent therapy for immuno-deficient diseases (such as AIDS), various types of cancer, viral infections and osteopetrosis has been established, it would be highly advantageous to have GcMAF composition devoid of protein contamination, suitable for intravenous administration.

### SUMMARY OF THE INVENTION

The present invention relates to therapeutic Gc-protein derived macrophage activating factor (GcMAF) compositions and methods of producing same.

The teachings of the present invention overcomes the shortages of hitherto disclosed GcMAF compositions, which were found to contain residues of the glycosidase enzymes used for transforming Gc protein to macrophage activating factor, and thus may cause adverse effects when administered pharmaceutically, particularly when administered intravenously.

The present invention is based in part on the unexpected finding that GcMAF preparations obtained from contacting Gc protein source with β-galactosidase immobilized on polygalactose-based resins (e.g. sepharose beads), contained deleterious amounts of the β-galactosidase enzyme. Without wishing to be bound by any specific theory or mechanism of action, the presence of the glycosidase residues found in the GcMAF compositions may be due to the phenomenon of solid support digestion by the glycosidase enzymes attached thereto.

Thus, according to one aspect, the present invention provides a composition comprising Gc protein-derived macrophage activating factor (GcMAF) and less than 0.5 % glycosidase enzymes out of the total protein content of the composition, wherein the GcMAF is produced by a process comprising the steps of: (a) contacting Gc protein in vitro with the glycosidase enzyme β-galactosidase or with β-galactosidase in combination with at least one additional glycosidase enzyme, wherein each of the glycosidase enzymes being immobilized on a solid phase devoid of the enzyme substrate; and (b) removing the immobilized enzyme from the GcMAF composition.

As used herein the term "essentially devoid of glycosidase enzymes" refers to a composition containing less than 3% glycosidase enzymes of the composition total protein content, typically less than 2% or 1%, more typically less than 0.5% or 0.2% of the total protein content of said composition.

According to certain embodiments, the GcMAF comprises vitamin D-binding protein (Gc Protein) or an active fragment thereof having an N-acetylgalactosamine group linked to an amino acid residue.

The terms "vitamin D-binding protein" and "Gc protein" are used herein interchangeably and refer to all the polymorphic forms of the glycoprotein and genetic variations thereof, including Gc2, Gc1, and the subtypes Gc1f, Gc1s and Gc1s*. As used herein the term "an active fragment thereof" refers to any part of Gc protein having a terminal N-acetylgalactosamine group linked to an amino acid residue, capable of macrophage activation.

According to certain embodiments, the Gc protein fragment comprises the amino acid sequence corresponding to amino acids 400-435 of all mature Gc protein polymorphic forms. According to other embodiments, the Gc fragment is Gc protein domain III corresponding to amino acids 375-458 of the mature protein.

According to certain embodiments, the Gc protein comprises the amino acid sequence selected from the group consisting of SEQ ID NOs:1-3 (Gc1f, Gc1s and Gc2, respectively). According to these embodiments, the N-acetylgalactosamine group is linked to the amino acid threonine at position 418 or amino acid threonine at position 420 of the mature Gc protein.

According to other embodiments, the Gc fragment Domain III, corresponding to amino acids 375-458 of the mature Gc protein consist of the amino acid sequence set forth in either SEQ ID NO:4 or SEQ ID NO:5. According to this embodiment, the N-acetylgalactosamine is linked to the amino acid threonine at position 44 or amino acid threonine at position 46.

According to certain embodiments, the isolated vitamin D-binding protein or any part thereof is purified from human blood serum. According to other embodiments, the isolated vitamin D-binding protein or its small domain responsible for macrophage activation can be produced from cloned polynucleotides employing recombination systems.

Removal of the glycosidase enzymes transforming the Gc protein to macrophage activating factor from the composition medium may be performed by any method as is known to a person skilled in the art, including, but not limited to, affinity chromatography, ion-exchange chromatography and gel filtration. According to certain typical embodiments, the enzymes are immobilized on a solid phase, particularly a solid phase devoid of the enzyme substrate.

Immobilizing the enzymes on a solid support has several advantages, including simple separation of the enzymes from the desired protein(s). The present invention now discloses that the use of solid phase devoid of the enzyme substrate prevents release of soluble enzyme into the reaction medium, facilitating the removal of the non-desired enzymes from the final composition.

Thus, according to additional aspect, the present invention provides a process for producing a GcMAF composition comprising less than 0.5 %glycosidase enzymes, the process comprising (a) contacting Gc protein or an active fragment thereof *in vitro* with the glycosidase enzyme β-galactosidase or with β-galactosidase in combination with at least one additional glycosidase enzyme, wherein each of the glycosidase enzymes is immobilized on a solid phase devoid of said enzyme substrate, to obtain Gc-macrophage activating factor (GcMAF); and (b) removing the immobilized enzyme from the GcMAF composition, thereby obtaining a GcMAF composition comprising less than 0.5 % glycosidase enzymes.

According to certain embodiments, the additional glycosidase enzyme is selected from the group consisting of mannosidase and sialidase.

According to certain embodiment, the process further comprises subjecting the GcMAF containing composition to ion exchange chromatography. According to typical embodiments, the chromatography is anion exchange chromatography, using, for examples Q-sepharose column. According to alternative embodiments, the process further comprises subjecting the GcMAF composition to hydrophobic interaction chromatography, for example phenyl sepharose column. According to other embodiment, the process further comprises combination of ion exchange chromatography and hydrophobic interaction chromatography.

According to certain embodiments, the β-galactosidase is immobilized on a solid phase comprising acrylic beads. According to particular embodiments, the solid phase is hydrophilic acrylic beads.

According to additional aspect, the present invention provides a pharmaceutical composition comprising a therapeutically effective amount the macrophage activating factor of the present invention, further comprising pharmaceutically acceptable diluent or carrier. According to preferred embodiments, the pharmaceutical composition is formulated for intravenous administration.

According to yet further aspect the present invention provides the above compositions for use in inducing macrophage activation in an individual in need thereof.

According to yet additional aspect, the present invention provides the above compositions for use in treating cancer or HIV-infected patients.

The GcMAF may be administered at a dose of 100-500ng/injection

According to certain embodiments, the cancer is associated with elevated levels of alpha-N-acetylgalactosaminidase (Nagalase). According to other embodiments, the cancer is selected from the group consisting of breast cancer, prostate cancer, colorectal cancer, liver cancer, lung cancer, head/neck cancer, brain cancer, kidney cancer, bladder cancer, stomach cancer, uterus cancer, ovarian cancer, skin cancer, fibrosarcoma, mesothelioma, leukemia and melanoma.

Other objects, features and advantages of the present invention will become clear from the following description and drawings.

### DETAILED DESCRIPTION OF THE INVENTION

The present invention provides therapeutic compositions comprising Gc protein-derived macrophage activating factor. The compositions of the invention are advantageous over hitherto known compositions, being devoid of contaminating enzymes and suitable for intravenous administration.

Gc protein, also designated vitamin D binding protein, is a serum factor comprising a polypeptide with specific oligosaccharides attached thereto. Step-wise removal of certain oligosaccharides with specific glycosidase enzymes results in transforming the Gc protein to highly potent macrophage activating factor (MAF) as disclosed in U.S. Patent No. 5,177,002 to the inventor of the present invention.

Human Gc protein can be purified from blood serum by any method as is known to a person skilled in the art. Gc protein of high purity for use in the process of the invention can be isolated from blood serum using 25-hydroxyvitamin D₃-Sepharose affinity chromatography according to the procedure of Link et al. (1986. Anal. Biochem. 157:262). The Gc protein may also be purified by actin-agarose affinity chromatography according to the procedure of Haddad et al. (1984. Biochem. J. 218:805), which takes advantage of the binding specificity of Gc protein for actin.

Alternatively, the Gc protein can be obtained from cloned cDNA encoding the human Gc protein or Gc protein small domain (domain III). Cloning and expression of Gc protein and Gc domain III was described in U.S. Patent No. 6,410,269 to the inventor of the present invention. The method described therein employs a human liver cDNA library in bacteriophage λgt11 (Clontech, Palo Alto, CA) for isolating a full length cDNA encoding the human Gc protein, and the use of the baculoviral expression system in insect cells for the protein expression. However, it is to be explicitly understood that any method/system as is known in the art can be used for expressing a cDNA encoding the Gc protein or active part thereof, including bacterial, insect and mammalian cell systems. Expression can be performed in a eukaryotic cell such that the Gc protein or its active domain is correctly glycosylated. Any such cell system known in the art may be used, for example Chinese hamster ovary (CHO) cells, BHK cells, human embryonic kidney HEK293 cells and Saccharomyces cerevisiae. Accordingly, any eukaryotic expression vector can be used, including, but not limited to, pCI-NEO, pWE3, pcDNA3.1 and pCM182. Insertion of the vector into the selected cell system can be performed, for example, by electroporation, lipids such as TransFectin or chemical methods as is known to a person skilled in art, with or without amplification. The transfection may result is transient or stable expression, both forms being adequate to obtain the desired Gc protein or part thereof. The expressed protein, being the precursor of active MAF according to the teaching of the present invention, can then be extracted from the cells or collected from the growth media by any method known in the art.

The term "Gc protein" or vitamin D-binding protein" as used herein refer to all genotypes, including Gc2, Gc1, and the subtypes Gc1f, Gc1s and Gc1s* and active variants and fragments thereof. "Active" Gc protein or fragment thereof as used herein refers to Gc protein capable of activating macrophages, particularly to Gc protein or a fragment thereof having an N-acetylgalactosamine group linked to an amino acid residue.

According to certain embodiments, the Gc protein comprises the amino acid sequence selected from the group consisting of SEQ ID NOs:1-3 (Gc1f, Gc1s and Gc2, respectively). According to these embodiments, the N-acetylgalactosamine group is linked to the amino acid threonine at position 418 or amino acid threonine at position 420 of the mature Gc protein.

According to certain embodiments, the Gc protein fragment comprises the amino acid sequence corresponding to amino acids 400-435 of all mature Gc protein polymorphic forms. According to other embodiments, the Gc fragment is Gc protein domain III corresponding to amino acids 375-458 of the mature protein.

According to other embodiments, the Gc fragment Domain III, corresponding to amino acids 375-458 of the mature Gc protein consist of the amino acid sequence set forth in either SEQ ID NO:4 or SEQ ID NO:5. According to this embodiment, the N-acetylgalactosamine is linked to the amino acid threonine at position 44 or amino acid threonine at position 46.

Macrophages have a potential to eliminate cancerous cells and HIV-infected cells when activated. Deglycosylation of GcMAF by the enzyme alpha-N-acetylgalactosaminidase (Nagalase) prevents it from activating macrophages and therefore suppresses the cell immune response. In HIV infected patients it has been suggested that defective antigen presentation is a factor in immune deficiency. The presence of elevated Nagalase in the plasma of HIV patients suggests that macrophage activation may be inhibited in these patients. In addition, Nagalase has been shown to be an intrinsic component of an envelope protein promoting fusion for the initiation of infection. The plasma concentration of Nagalase in patients with systemic lupus erythematosus was also found to be elevated. In lupus, autoantibodies form pathogenic immune complexes and are deposited in tissues. The clearance of these complexes by macrophages is inhibited if macrophage activation is disrupted. Cancer cells have been shown to produce Nagalase and elevated concentrations in serum have been recorded in a number of cancer patients suffering from melanoma, prostate, colorectal, and metastatic breast cancer. Administering exogenous GcMAF to such patients may thus overcome the shortage in active macrophages due to the elevated concentrations of Nagalase. Indeed, GcMAF has been shown to act directly and activate macrophages or osteoclasts of cancer, HIV-infected and osteoperotic patients, and preliminary clinical trails showed a curative effect of GcMAF on several types of human cancer (e.g. Pacini S. et al., 2012. Anticancer Res.;32(1):45-52; Gregory KJ et al. 2010. PLoS One. 2010 5(10):e13428).

To be suitable for pharmaceutical use, particularly when formulated for intravenous administration, the GcMAF composition should stand meticulous requirements of being non-toxic and highly tolerable by human.

Accordingly, the glycosidase enzymes required for transforming Gc protein or Domain III thereof to active MAF as well as potential other contaminant should be removed from the MAF composition.

According to one aspect, the present invention provides a composition comprising Gc protein-derived macrophage activating factor (GcMAF) and less than 0.5 % glycosidase enzymes out of the total protein content of the composition, wherein the GcMAF is produced by a process comprising the steps of: (a) contacting Gc protein in vitro with the glycosidase enzyme β-galactosidase or with β-galactosidase in combination with at least one additional glycosidase enzyme, wherein each of the glycosidase enzymes being immobilized on a solid phase devoid of the enzyme substrate; and (b) removing the immobilized enzyme from the GcMAF composition.

According to additional aspect, the present invention provides a process for producing a GcMAF composition essentially devoid of glycosidase enzymes, the process comprising (a) contacting Gc protein or an active fragment thereof in vitro with the glycosidase enzyme β-galactosidase or with β-galactosidase in combination with at least one additional glycosidase enzyme, wherein each of the glycosidase enzymes is immobilized on a solid phase devoid of said enzyme substrate, to obtain Gc-macrophage activating factor (GcMAF); and (b) removing the immobilized enzyme from the GcMAF composition, thereby obtaining a GcMAF composition essentially devoid of glycosidase enzymes.

Removal of the glycosidase enzymes may be performed by any method as is known in the art. According to certain typical embodiments of the invention, the Gc protein or part thereof is contacted with the enzyme(s) wherein the enzymes are immobilized on a solid phase, wherein the solid phase is devoid of the enzyme substrate. Of particular importance is the immobilization of β-galactosidase on a solid phase devoid of galactose. The present invention now discloses that β-galactosidase is released from the sepharose solid phase during the incubation time required for activating Gc protein to MAF. Thus, while in the hitherto disclosed process the immobilized β-galactosidase is removed from the composition, the soluble enzyme or residues thereof can still contaminate the composition.

The compositions of the present invention comprising GcMAF comprise less than 0.5% glycosidase enzymes out of the total protein content of said composition. As used herein, the term "percentage (%) of the total protein of the total protein content" refers to the weight/weight percentage of the glycosidase enzymes out of the total protein content.

According to certain embodiments, the immobilization solid phase of β-galactosidase which, according to the teaching of the present invention is devoid of the galactosidase substrate is composed of acrylic beads, capable of conjugating the enzyme. Several commercially available resins may be used for immobilization of the galactosidase enzymes, including, but not limited to Profinity epoxide (BioRad); Fractogel® Epoxy (Merck); and Eupergit® (BioChemika, Sigma).

In addition to β-galactosidase, at least one additional glycosidase enzyme may be used, depending on the Gc protein genotype. For activating Gc1f protein, β-galactosidase and sialidase are used to convert Gc1f to GcMAF. For activating Gc1s protein, β-galactosidase and mannosidase are used to convert Gc1s to GcMAF. As for β-galactosidase, each of the additional enzymes used may be immobilized on a solid phase. To avoid the presence of mannosidase and/or sialidase in the final composition according to the teachings of the present invention, each of the enzymes mannosidase and sialidase is immobilized on a substrate devoid of the enzyme substrate.

The mannosidase or sialidase can be immobilized on beads based on agarose.

Alternatively and additionally, the glycosidase enzymes, either soluble or immobilized, are removed from the GcMAF composition by several chromatography techniques and/or filtration and/or precipitation and/or centrifugation, separating the enzymes from the desired GcMAF protein.

Typical chromatography techniques include separation by size (size exclusion, also referred to as gel filtration); by charge (ion exchange chromatography), by hydrophobicity (hydrophobic interaction chromatography and reverse phase chromatography) and by bio-recognition (affinity chromatography).

According to certain embodiments, the process further comprises subjecting the GcMAF containing composition to ion exchange chromatography. According to typical embodiments, the chromatography is anion exchange chromatography. Various types of anion exchange resins can be used, including DEAE-Sephadex, QAE-Sephadex, DEAE-Sephacel, DEAE-cellulose, DEAE-Sepharose, Q-sepharose and the like.

The present invention now discloses that subjecting the GcMAF composition to anion exchange column further results in the separation of active GcMAF from non-active forms. Thus, the present invention provides means for achieving high yield isolation and purification of active GcMAF from various sources.

The anion exchange resin may be Q-Sepharose. Variety of conditions may be used in this particular step. The anion exchange resin can first be equilibrated with buffer solution having a pH between 4.5-9.5 and a conductivity of below 12.0 mS/cm. After the resin is equilibrated, the fraction containing GcMAF is adjusted to ion strength below 12 mS/cm by dilution and loaded on the anion exchange resin. These conditions of pH and conductivity allow the retention of GcMAF on the column, while the anion exchange medium is washed. The conductivity of the washing buffer (at a pH range of from 4.5 to 9.5) is increased during the washing. This increase provides suitable conditions such that no GcMAF is discarded in the flow through, to give maximal GcMAF yield.

The GcMAF is then eluted from the column. Elution can be performed with a buffer solution having a pH between 4.5-9.5 and conductivity greater than 3mS/cm.

According to other embodiments, the process further comprises subjecting the GcMAF composition to hydrophobic interaction chromatography, for example phenyl sepharose column.

The hydrophobic interaction resin may be phenyl-Sepharose. Variety of conditions may be used in this particular step. The hydrophobic interaction resin may first be equilibrated with buffer solution having a pH between 4.5-9.5 and a conductivity of above 15.0 mS/cm. After the resin is equilibrated, the fraction containing GcMAF is adjusted to ion strength above 15.0 mS/cm by dilution and loaded on the hydrophobic interaction resin. These conditions of pH and conductivity allow the retention of GcMAF on the column, while the hydrophobic interaction medium is washed. The conductivity of the washing buffer (at a pH of between 4.5-9.5) is decreased during the washing. This decrease provides suitable conditions such that no GcMAF is discarded in the flowthrough, to give maximal GcMAF yield.

The GcMAF is then eluted from the column. Elution can be performed with a buffer solution having a pH between 4.5-9.5 and conductivity below 25mS/cm.

The sterility of the compositions of the present invention is of major concern, as the product should be administered to humans for therapeutic purposes, in particular by intravenous administration. Although the serum source material is typically examined for the presence of contaminating viruses and a great effort is taken to exclude contaminated donor fractions, there is a need to further assure that the end product of the process would be virus-free.

Accordingly, the process of the present invention can further comprises viral removal and/or viral inactivation steps. Viral reduction can be accomplished by several processes, including nanofiltration; solvent/detergent treatment; iodine inactivation, e.g., treatment with an iodinated ion exchange matrix material such as iodinated SEPHADEX™ (as disclosed in PCT applications WO 97/48422 and WO 97/48482); treatment with Pathogen Inactivating Compounds; heat inactivation, gamma irradiation; or any other suitable virucidal process.

Lipid coated viruses are effectively inactivated by treatment with non-ionic biocompatible solvents and detergents. Methods for virus inactivation by solvent-detergent applications are described, for example, in EP 0131740. However, non-lipid coated viruses cannot be inactivated by solvent-detergent treatments, thus, other inactivation methodologies have to be used for their inactivation, including eliminating by physical means, e.g., the filtration of the composition through very small pore size filter so as to remove viruses by size exclusion (nanofiltration).

Following separation of the glycosidase enzymes from the active GcMAF and viral removal, the solution can be treated to reduce its water content and change the ionic composition by conventional means such as by diafiltration, ultrafiltration, lyophilization, etc., or combinations thereof.

The composition comprising the purified GcMAF can be dialyzed against PBS buffer by ultrafiltration with MWCO between 5-50 kDa. The GcMAF protein is then diluted with PBS to its final concentration and is filtered through 0.1 or 0.2 µm membrane to obtain sterile GcMAF composition.

According to additional aspect, the present invention provides a pharmaceutical composition comprising a therapeutically effective amount of the macrophage activating factor of the present invention, further comprising pharmaceutically acceptable diluent or carrier. According to preferred embodiments, the pharmaceutical composition is formulated for intravenous administration.

As used herein, the term "therapeutically effective amount" refers to an amount of a protein or protein formulation or composition which is effective to treat a condition in a living organism to whom it is administered over some period of time.

Pharmaceutical compositions of the present invention may be manufactured by processes well known in the art, e.g. by means of conventional mixing, dissolving, granulating, grinding, pulverizing, dragee-making, levigating, emulsifying, encapsulating, entrapping or lyophilizing processes. The purpose of a pharmaceutical composition is to facilitate administration of a compound to an organism.

Pharmaceutical compositions for use in accordance with the present invention thus may be formulated in conventional manner using one or more acceptable diluents or carriers comprising excipients and auxiliaries, which facilitate processing of the active compounds into compositions, which can be used pharmaceutically. Proper formulation is dependent on the route of administration chosen. According to typical embodiment the pharmaceutical compositions of the present invention are formulated for intravenous administration.

For intravenous injection, the compounds of the invention may be formulated in aqueous solutions, preferably in physiologically compatible buffers such as Hank's solution, Ringer's solution, or physiological saline buffer. Aqueous injection suspensions may contain substances, which increase the viscosity of the suspension, such as sodium carboxymethyl cellulose, sorbitol or dextran. Optionally, the suspension may also contain suitable stabilizers or agents to increase the stability and solubility of the compounds and to allow for compositions of highly concentrated solutions.

Alternatively, the active ingredient may be in powder form for reconstitution with a suitable vehicle, e.g., sterile, pyrogen-free water, before use.

Pharmaceutical compositions suitable for use in context of the present invention include compositions wherein the active ingredients are contained in an amount effective to achieve the intended purpose. More specifically, a therapeutically effective amount means an amount of a compound effective to prevent, alleviate or ameliorate symptoms of a disease of the subject being treated.

Determination of a therapeutically effective amount is well within the capability of those skilled in the art. The GcMAF is typically administered at a dose of 100-500 ng/injection. A skilled artisan can determine the regime of administration according to parameters associated with the particular disease and stage to be treated as well as on characteristics of the treated individual (age, size, gender, etc.).

Toxicity and therapeutic efficacy of the compounds described herein can be determined by standard pharmaceutical procedures in cell cultures or experimental animals, e.g., by determining the IC50 (the concentration which provides 50% macrophage activation) and the LD50 (lethal dose causing death in 50 % of the tested animals) for a GcMAF compound according to the present invention. The data obtained from these cell culture assays and animal studies can be used in formulating a range of dosage for use in human. The dosage may vary depending upon the dosage form employed and the route of administration utilized. The exact formulation, route of administration and dosage can be chosen by the individual physician in view of the patient's condition. (See e.g., Fingl, et al., 1975, in "The Pharmacological Basis of Therapeutics", Ch. 1 p.1).

Depending on the severity and responsiveness of the condition to be treated, dosing can be determined. The pharmaceutical composition comprising the GcMAF of the invention can be administered via intravenous injection once a week for several weeks. The dosing may also be a single administration of a slow release composition, with course of treatment lasting from several days to several weeks or until cure is affected or diminution of the disease state is achieved.

The amount of a composition to be administered will, of course, be dependent on the subject being treated, the severity of the affliction, the manner of administration, the judgment of the prescribing physician, etc.

The following example is presented in order to more fully illustrate some embodiments of the invention. It should, in no way be construed, however, as limiting the broad scope of the invention. One skilled in the art can readily devise many variations and modifications of the principles disclosed herein without departing from the scope of the invention.

### EXAMPLES

### Example 1: GcMAF production

### Step 1: Enrichment - precipitation with Ammonium sulfate

Blood was obtained from healthy donors. Gc protein type of each blood sample was determined using specific primers in a PCR reaction and further by mass spectrometry. Typing was performed by BioGlobe GmbH Hamburg, Germany, based on the method described in Abbas et al. 2008 (Cancer Epidemiol Biomarkers Prev 17:1339-1343). Samples of donors homozygous to the Gc1f allele were taken for further processing. One out of 50 samples screened in 2009 and seven out of 105 samples screened in 2010 were identified as homozygous for Gc1f.

The serum fraction was isolated from the blood samples, and the Gc protein was precipitated from serum by 70% ammonium sulfate (AS). After centrifugation, the precipitated protein was dissolute in PBS and dialyzed against the same buffer having pH about 7.0-7.6.

### Step 2: Capture - Vitamin D-sepharose affinity column

The protein was loaded on a 25-OH-vitamin D affinity column pre-equilibrated with TEST buffer (Tris, EDTA, Saline, Triton, pH 7.4). After washing with TEST buffer the protein was eluted by 6M GuHCl. Fractions with peak absorbance at 280nm were pooled and dialyzed against a phosphate buffer having pH 7.0.

### Step 3: Purification - hydroxyapetite column

The protein was loaded on a hydroxyapatite column pre-equilibrated with phosphate buffer having pH 7.0. After washing with the same buffer the protein was eluted by a 10-200mM linear gradient of phosphate. Fractions containing the protein were pooled.

### Step 4: Gc activation (preparation of GcMAF)

The pooled protein fraction, containing Gc1f type only, was treated with β-galactosidase conjugated to acrylic beads (Profinity Epoxide, BioRad) for 1 hour in PBS pH 7.4 at 37°C with gentle mixing. The enzyme conjugated to the acrylic beads was removed by centrifugation, and the protein fraction was collected. Next, the protein fraction was treated with Neuraminidase conjugated to agarose for 1 hour in 0.1M NaAc 2mM CaCl₂ pH 5.0 at 37°C with gentle mixing and the conjugated enzyme was removed by centrifugation. The protein fraction, now containing GcMAF, was collected and filtrated through a 0.22µm filter and stored at 4°C. The protein type was verified by ELISA and it quantity by Bradford assay.

### Step 5: Final purification

GcMAF is processed on Ion Exchange (IEX) column (such as Q sepharose) and on Hydrophobic interaction chromatography (HIC) column (such as phenyl sepharose) to remove residual contaminating enzymes and non activated Gc protein. The composition is then examined to be essentially devoid of glycosidase enzymes by measuring glycosidase activity and/or presence of glycosidase using specific antibodies.

### Step 6: Formulation

The purified protein is dialyzed against PBS by ultrafiltration with MWCO between 10kDa and 50kDa. The protein is then diluted with PBS to its final concentration and filtered through 0.2 µm Millipore filter for sterilization.

## Claims

1. A composition comprising Gc protein-derived macrophage activating factor (GcMAF) and less than 0.5 % glycosidase enzymes out of the total protein content of the composition, wherein the GcMAF is produced by a process comprising the steps of: (a) contacting Gc protein *in vitro* with the glycosidase enzyme β-galactosidase or with β-galactosidase in combination with at least one additional glycosidase enzyme, wherein each of the glycosidase enzymes being immobilized on a solid phase devoid of the enzyme substrate; and (b) removing the immobilized enzyme from the GcMAF composition.

2. The composition of claim 1, wherein the GcMAF comprises vitamin D-binding protein (Gc Protein) or a fragment thereof having an N-acetylgalactosamine group linked to an amino acid residue.

3. The composition of claim 2,
wherein the Gc protein fragment comprises the amino acid sequence corresponding to amino acids 400-435 of the Gc Protein; or
wherein the GcMAF comprises the amino acid sequence set forth in any one of SEQ ID NOs:1-3, preferably,
wherein the N-acetylgalactosamine group is linked to the amino acid threonine at a position selected from the group consisting of position 418 and position 420; or
wherein the Gc fragment consists of the amino acid sequence set forth in SEQ ID NO:4 or SEQ ID NO:5; preferably wherein the N-acetylgalactosamine group is linked to the amino acid threonine at a position selected from the group consisting of position 44 and position 46.

4. The composition of any one of claims 1-3,
wherein the Gc-protein or fragment thereof is purified from blood serum or plasma; or
wherein the Gc-protein or fragment thereof is produced from a cloned polynucleotide.

5. A process for producing a GcMAF composition comprising less than 0.5 % glycosidase enzymes out of the total protein content of the composition, the process comprising
(a) contacting Gc protein or an active fragment thereof *in vitro* with the glycosidase enzyme β-galactosidase or with β-galactosidase in combination with at least one additional glycosidase enzyme, wherein each of the glycosidase enzymes is immobilized on a solid phase devoid of said enzyme substrate, to obtain Gc-macrophage activating factor (GcMAF); and
(b) removing the immobilized enzyme from the GcMAF, thereby obtaining a GcMAF composition comprising less than 0.5 % glycosidase enzymes out of the total protein content of the composition.

6. The process of claim 5,
wherein said additional glycosidase enzyme is selected from the group consisting of mannosidase and sialidase; or
wherein the GcMAF comprises Gc Protein or a fragment thereof having an N-acetylgalactosamine group linked to an amino acid residue, preferably wherein the Gc protein comprises the amino acid sequence set forth in any one of SEQ ID NOs:1-3 or a fragment thereof, more preferably wherein the N-acetylgalactosamine group is linked to the amino acid threonine at a position selected from the group consisting of position 418 and position 420; or
wherein the Gc protein fragment comprises the amino acid sequence corresponding to amino acids 400-435 of the Gc Protein, more preferably wherein the Gc protein fragment consists of the amino acids sequence set forth in SEQ ID NO:4 or SEQ ID NO:5, even more preferred wherein the N-acetylgalactosamine group is linked to the amino acid threonine at a position selected from the group consisting of position 44 and position 46.

7. The process of any one of claims 5-6,
wherein the Gc-protein or fragment thereof is purified from blood serum or plasma; or wherein the Gc-protein or fragment thereof is produced from a cloned polynucleotide.

8. The process of claim 5,
further comprising subjecting the GcMAF containing composition to ion exchange chromatography, preferably comprising subjecting the GcMAF containing composition to anion exchange chromatography; or
further comprising subjecting the GcMAF containing composition to hydrophobic interaction chromatography; or
further comprising subjecting the GcMAF containing composition to a combination of ion exchange chromatography and hydrophobic interaction chromatography; or
wherein the β- galactosidase is immobilized on a solid phase comprising acrylic beads.

9. A pharmaceutical composition comprising a therapeutically effective amount of a composition comprising
macrophage activating factor according to claim 1, further comprising a therapeutically acceptable diluent or carrierr.

10. The pharmaceutical composition of claim 9, formulated for intravenous administration.

11. A composition of claim 10 for use in a method
for inducing macrophage activation in an individual in need thereof; or
for treating cancer; or
for treating an HIV-infected patient.

12. The composition of claim 11 for use in a method for treating cancer or for treating an HIV infected patient, wherein the cancer or HIV is associated with elevated levels of alpha-N-acetylgalactosaminidase (Nagalase), preferably wherein the cancer is selected from the group consisting of breast cancer, prostate cancer, colorectal cancer, liver cancer, lung cancer, head/neck cancer, brain cancer, kidney cancer, bladder cancer, stomach cancer, uterus cancer, ovarian cancer, skin cancer, fibrosarcoma, mesothelioma, leukemia and melanoma.

## Patentansprüche

1. Zusammensetzung umfassend vom Gc Protein abstammenden Makrophagenaktivierungsfaktor (GcMAF) und weniger als 0,5% Glycosidaseenzyme an dem Gesamtproteingehalt der Zusammensetzung, wobei der GcMAF durch ein Verfahren hergestellt wurde umfassend die Schritte: (a) in Kontakt bringen des Gc Proteins *in vitro* mit dem Glycosidaseenzym β-Galactosidase oder mit β-Galactosidase in Verbindung mit wenigstens einem zusätzlichem Glycosidaseenzym, wobei jedes der Glycosidaseenzyme an einer festen Phase immobilisiert ist, die frei von dem Enzymsubstrat ist, und (b) Entfernen des immobilisierten Enzyms aus der GcMAF Zusammensetzung.

2. Zusammensetzung nach Anspruch 1, worin das GcMAF Vitamin D-Bindungsprotein (Gc Protein) oder ein Fragment davon umfasst, das eine N-Acetylgalactosamingruppe aufweist, die mit einem Aminosäurerest verbunden ist.

3. Zusammensetzung nach Anspruch 2,
worin das Gc Proteinfragment die Aminosäuresequenz umfasst, die den Aminosäuren 400-435 des Gc Proteins entspricht,
worin das GcAMF die Aminosäuresequenz umfasst, die in einer der SEQ ID NR: 1-3 aufgeführt ist, vorzugsweise
worin die N-Acetylgalactosamingruppe mit der Aminosäure Threonin an einer Position verbunden ist, die aus der Gruppe bestehend aus Position 418 und Position 420 ausgewählt ist, oder
worin das Gc Fragment aus der Aminosäuresequenz besteht, die in der SEQ ID NR: 4 oder SEQ ID NR: 5 aufgeführt ist, vorzugsweise worin die N-Acetylgalactosamingruppe mit der Aminosäure Threonin an einer Position verbunden ist, die aus der Gruppe bestehend aus Position 44 und Position 46 ausgewählt ist.

4. Zusammensetzung nach einem der Ansprüche 1 bis 3,
worin das Gc Protein oder das Fragment davon aus Blutserum oder Plasma aufgereinigt wurde, oder
worin das Gc Protein oder das Fragment davon aus einem klonierten Polynukleotid hergestellt wurde.

5. Verfahren zur Herstellung einer GcAMF Zusammensetzung umfassend weniger als 0,5% Glycosidaseenzyme an dem Gesamtproteingehalt der Zusammensetzung, wobei das Verfahren umfasst:
(a) in Kontakt bringen des Gc Proteins oder eines aktiven Fragments davon *in vivo* mit dem Glycosidaseenzym β-Galactosidase oder mit β-Galactosidase in Verbindung mit wenigstens einem zusätzlichem Glycosidaseenzym, wobei jedes der Glycosidaseenzyme an einer festen Phase immobilisiert ist, die frei von dem Enzymsubstrat ist, um den vom Gc Protein abstammenden Makrophagenaktivierungsfaktor (GcMAF) zu erhalten, und
(b) Entfernen des immobilisierten Enzyms von dem GcMAF, dabei Erhalten einer GcAMF Zusammensetzung umfassend weniger als 0,5% Glycosidaseenzyme an dem Gesamtproteingehalt der Zusammensetzung.

6. Verfahren nach Anspruch 5,
wobei das zusätzliche Glycosidaseenzym ausgewählt ist aus der Gruppe bestehend aus Mannosidase und Sialidase, oder
wobei das GcMAF das Gc Protein oder ein Fragment davon umfasst, das eine N-Acetylgalactosamingruppe aufweist, die mit einem Aminosäurerest verbunden ist, vorzugsweise wobei das Gc Protein die Aminosäuresequenz umfasst, die in einer der SEQ ID NR: 1-3 aufgeführt ist oder ein Fragment davon, mehr bevorzugt wobei die N-Acetylgalactosamingruppe mit der Aminosäure Threonin an einer Position verbunden ist, die aus der Gruppe bestehend aus Position 418 und Position 420 ausgewählt ist, oder
wobei das Gc Proteinfragment die Aminosäuresequenz umfasst, die den Aminosäuren 400-435 des Gc Proteins entspricht, mehr bevorzugt wobei das Gc Proteinfragment aus den Aminosäuresequenzen besteht, die in der SEQ ID NR: 4 oder SEQ ID NR: 5 aufgeführt sind, noch mehr bevorzugt wobei die N-Acetylgalactosamingruppe mit der Aminosäure Threonin an einer Position verbunden ist, die aus der Gruppe bestehend aus Position 44 und Position 46 ausgewählt ist.

7. Verfahren nach einem der Ansprüche 5 bis 6,
wobei das Gc Protein oder das Fragment davon aus Blutserum oder Plasma aufgereinigt wurde, oder
wobei das Gc Protein oder das Fragment davon aus einem klonierten Polynukleotid hergestellt wurde.

8. Verfahren nach Anspruch 5,
ferner umfassend ein Aussetzen der GcAMF enthaltenen Zusammensetzung einer Ionenaustauschchromatographie, vorzugsweise Aussetzen der GcAMF enthaltenen Zusammensetzung einer Anionenaustauschchromatographie, oder
ferner umfassend ein Aussetzen der GcAMF enthaltenen Zusammensetzung einer hydrophoben Interaktionschromatographie, oder
ferner umfassend ein Aussetzen der GcAMF enthaltenen Zusammensetzung einer Kombination aus Ionenaustauschchromatographie und hydrophober Interaktionschromatographie, oder
wobei die β-Galctosidase auf einer festen Phase immobilisiert ist, die Acrylbeads umfasst.

9. Pharmazeutische Zusammensetzung umfassend eine therapeutisch wirksame Menge einer Zusammensetzung umfassend Makrophagenaktivierungsfaktor nach Anspruch 1, ferner umfassend ein therapeutisch verträgliches Verdünnungsmittel oder Träger.

10. Pharmazeutische Zusammensetzung nach Anspruch 9, formuliert für eine intravenöse Verabreichung.

11. Zusammensetzung nach Anspruch 10 zur Verwendung in einem Verfahren
zur Induktion einer Makrophagenaktivierung in einem Individuum mit Bedarf dafür, oder
zur Behandlung von Krebs, oder
zur Behandlung eines mit HIV infizierten Patienten.

12. Zusammensetzung nach Anspruch 11 zur Verwendung in einem Verfahren zur Behandlung von Krebs oder zur Behandlung eines mit HIV infizierten Patienten, wobei der Krebs oder HIV mit erhöhten Spiegeln von alpha-N-Acetylgalactosaminidase (Nagalase) einhergeht, vorzugsweise wobei der Krebs ausgewählt ist aus der Gruppe bestehend aus Brustkrebs, Prostatakrebs, kolorektalem Krebs, Leberkrebs, Lungenkrebs, Kopf/Nackenkrebs, Hirnkrebs, Nierenkrebs, Blasenkrebs, Magenkrebs, Gebärmutterkrebs, Eierstockkrebs, Hautkrebs, Fibrosarkom, Mesotheliom, Leukämie und Melanom.

## Revendications

1. Composition comprenant un facteur d'activation de macrophages dérivé d'une protéine Gc (GcMAF) et moins de 0,5 % d'enzymes glycosidases par rapport à la teneur en protéine totale de la composition, dans laquelle le GcMAF est produit selon un processus comprenant les étapes consistant à : (a) mettre en contact une protéine Gc *in vitro* avec l'enzyme β-galactosidase glycosidase ou avec la β-galactosidase en combinaison avec au moins une enzyme glycosidase supplémentaire, dans laquelle chacune des enzymes glycosidases étant immobilisée sur une phase solide dépourvue de substrat enzymatique ; et (b) éliminer l'enzyme immobilisée à partir de la composition de GcMAF.

2. Composition selon la revendication 1, dans laquelle le GcMAF comprend une protéine se fixant à la vitamine D (protéine Gc) ou un fragment de celle-ci possédant un groupe N-acétylgalactosamine lié à un résidu d'acide aminé.

3. Composition la revendication 2,
dans laquelle le fragment de protéine Gc comprend la séquence d'acides aminés correspondant aux acides aminés 400 à 435 de la protéine Gc ; ou
dans laquelle le GcMAF comprend la séquence d'acides aminés selon l'une quelconque des SEQ ID No. 1 à 3, de préférence,
dans laquelle le groupe N-acétylgalactosamine est lié à l'acide aminé thréonine au niveau d'une position choisie parmi le groupe constitué de la position 418 et de la position 420 ; ou
dans laquelle le fragment Gc consiste en la séquence d'acides aminés selon l'une de la SEQ ID No. 4 ou SEQ ID No. 5 ; de préférence dans laquelle le groupe N-acétylgalactosamine est lié à l'acide aminé thréonine au niveau d'une position choisie parmi le groupe constitué de la position 44 et de la position 46.

4. Composition selon l'une quelconque des revendications 1 à 3,
dans laquelle la protéine Gc ou le fragment de celle-ci est purifié à partir de sérum sanguin ou de plasma ; ou
dans laquelle la protéine Gc ou le fragment de celle-ci est produit à partir d'un polynucléotide cloné.

5. Processus de production d'une composition de GcMAF comprenant moins de 0,5 % d'enzymes glycosidases par rapport à la teneur de protéine totale de la composition, le processus consistant à
(a) mettre en contact une protéine Gc ou un fragment actif de celle-ci *in vitro* avec l'enzyme β-galactosidase glycosidase ou avec la β-galactosidase en combinaison avec au moins une enzyme glycosidase supplémentaire, dans laquelle chacune des enzymes glycosidases est immobilisée sur une phase solide dépourvue de substrat enzymatique, pour obtenir un facteur d'activation de macrophages dérivé d'une protéine Gc (GcMAF) ; et
(b) éliminer l'enzyme immobilisée depuis la composition de GcMAF, obtenant ainsi une composition de GcMAF comprenant moins de 0,5 % d'enzymes glycosidases par rapport à la teneur de protéine totale de la composition.

6. Processus selon la revendication 5,
dans lequel ladite enzyme glycosidase supplémentaire est choisie parmi le groupe constitué de la mannosidase et de la sialidase ; ou
dans lequel le GcMAF comprend la protéine Gc ou un fragment de celle-ci possédant un groupe N-acétylgalactosamine lié à un résidu d'acides aminés, de préférence dans lequel la protéine Gc comprend la séquence d'acides aminés selon l'une quelconque des SEQ ID No. 1 à 3 ou un fragment de celle-ci, plus préférablement dans lequel le groupe N-acétylgalactosamine est lié à l'acide aminé thréonine au niveau d'une position choisie parmi le groupe constitué de la position 418 et de la position 420 ; ou
dans lequel le fragment de protéine Gc comprend la séquence d'acides aminés correspondant aux acides aminés 400 à 435 de la protéine Gc, plus préférablement dans lequel le fragment de protéine Gc consiste en la séquence d'acides aminés selon l'une de la SEQ ID No. 4 ou SEQ ID No. 5 ; de préférence dans lequel le groupe N-acétylgalactosamine est lié à l'acide aminé thréonine au niveau d'une position choisie parmi le groupe constitué de la position 44 et de la position 46.

7. Processus selon l'une quelconque des revendications 5 à 6,
dans lequel la protéine Gc ou le fragment de celle-ci est purifié à partir de sérum sanguin ou de plasma ; ou
dans lequel la protéine Gc ou le fragment de celle-ci est produit à partir d'un polynucléotide cloné.

8. Processus selon la revendication 5,
consistant en outre à assujettir le GcMAF contenant la composition à une chromatographie échangeuse d'ions, de préférence consistant à assujettir le GcMAF contenant la composition à une chromatographie échangeuse d'anions ; ou
consistant en outre à assujettir le GcMAF contenant la composition à une chromatographie à interaction hydrophobe ; ou
consistant en outre à assujettir le GcMAF contenant la composition à une combinaison de chromatographie échangeuse d'ions et de chromatographie à interaction hydrophobe ; ou
dans lequel la β-galactosidase est immobilisée sur une phase solide comprenant des billes acryliques.

9. Composition pharmaceutique comprenant une quantité thérapeutiquement efficace d'une composition comprenant
un facteur d'activation de macrophages selon la revendication 1, comprenant en outre un diluant ou un véhicule pharmaceutiquement acceptable.

10. Composition pharmaceutique selon la revendication 9, formulée pour une administration intraveineuse.

11. Composition selon la revendication 10 pour une utilisation dans un procédé
destiné à induire une activation de macrophages chez un individu en ayant besoin ; ou
destiné au traitement du cancer ; ou
destiné au traitement d'un patient infecté par le VIH.

12. Composition selon la revendication 11 pour une utilisation dans un procédé destiné au traitement du cancer ou destiné au traitement d'un patient infecté par le VIH, dans laquelle le cancer ou le VIH est associé à des niveaux élevés d'alpha-N-acétylgalactosaminidase (Nagalase), de préférence dans laquelle le cancer est choisi parmi le groupe constitué du cancer du sein, du cancer de la prostate, du cancer colorectal, du cancer du foie, du cancer des poumons, du cancer de la tête et du cou, du cancer du cerveau, du cancer du rein, du cancer de la vessie, du cancer de l'estomac, du cancer de l'utérus, du cancer ovarien, du cancer de la peau, du fibrosarcome, du mésothéliome, de la leucémie et du mélanome.
